# EUROPÄISCHE PATENTSCHRIFT

(11) **EP 0 497 159 B1**
(45) Veröffentlichungstag und Bekanntmachung des Hinweises auf die Patenterteilung: **27.03.1996**
(21) Anmeldenummer: 92100706.8
(22) Anmeldetag: 17.01.1992
(51) Int. Cl.: A61M 3/02

(54) **Vorrichtung zur Spülung von Körpergefässen eines Patienten**
Device for irrigation of body cavities
Dispositif d'irrigation de cavités corporelles

(30) Priorität: 31.01.1991 DE 4102843
(43) Veröffentlichungstag der Anmeldung: 05.08.1992
(73) Patentinhaber: Fresenius AG, D-61350 Bad Homburg (DE)
(72) Erfinder: Meisberger, Arthur, W-6690 St. Wendel (DE); Kreutzig, Thomas, Dr. med., W-7801 Au bei Freiburg (DE)
(74) Vertreter: Dr. Fuchs, Dr. Luderschmidt Dr. Mehler, Dipl.-Ing. Weiss Patentanwälte

(56) Entgegenhaltungen:
- EP-A- 0 088 954
- FR-A- 2 285 149
- US-A- 4 261 360
- PATENT ABSTRACTS OF JAPAN vol. 15, no. 256 (C-845)28. Juni 1991 & JP-A-89 223484 (OLYMPUS OPTICAL)

## Beschreibung

Die Erfindung betrifft eine Vorrichtung zur Spülung von Körperhöhlen und Gewebeoberflächen eines Patienten.

Eine derartige Vorrichtung ist beispielsweise aus der US 3,570,488 bekannt. Nach Operationen, wie z.B. Prostataoperationen, wird mittels des Katheters dieser bekannten Vorrichtung die Blase des Patienten kontinuierlich z.B. mit Kochsalzlösung gespült, um bei Nachblutungen die Bildung von Blutgerinnseln zu vermeiden. Der Spülfluß wird bei der bekannten Vorrichtung mittels der Volumenstrom-Einstelleinrichtung von Hand eingestellt und von Zeit zu Zeit überprüft. Dies ergibt jedoch den Nachteil, daß der Spülfluß über lange Strecken nicht optimal ist. Besonders ungünstig ist daher das unerwartete Auftreten von verstärkten Blutungen, auf die dann nicht oder verspätet reagiert werden kann.

Eine Vorrichtung zum Spülen der im Oberbegriff des Patentanspruchs 1 angegebenen Art ist aus US-A-4,261,360 bekannt. Die bekannte Vorrichtung wird in Verbindung mit einem Resektoskop verwendet, um ein operierendes Organ, z.B. die Blase eines Patienten, mit einer Spülflüssigkeit zu füllen, so daß sich in der Blase ein bestimmter Druck aufbaut, wodurch sich die Blase aufweitet. Die Spülflüssigkeit wird über eine erste Pumpe gefördert und dem Einlaß des Resektoskops zugeführt. Der Ausgang des Resektoskops ist über eine vom Körper des Patienten wegführenden Leitung mit einer zweiten Pumpe zum Abpumpen der Flüssigkeit verbunden. Zwischen dem Auslaß der zweiten Pumpe und dem Reservoir zur Aufnahme der gebrauchten Spülflüssigkeit befindet sich eine Sensoreinrichtung. Mit der Sensoreinrichtung wird die Konzentration von Urin und Blut in der Spülflüssigkeit festgestellt, um die Förderrate der in der Auslaßleitung angeordneten Pumpe derart zu steuern, daß der Druck in der Blase des Patienten konstant bleibt.

Ferner ist aus EP-A-0 088 954 eine Vorrichtung zum Injizieren einer Versorgungslösung in eine Hirnkammer eines Patienten zur Behandlung eines Hirnschlags bekannt. Bei der bekannten Vorrichtung wird die Versorgungslösung unter Druck zugeführt, um anschließend wieder abgesaugt zu werden. Da die abgesaugte Flüssigkeit eine andere Zusammensetzung als die injizierte Versorgungslösung hat, kann sie zu diagnostischen Zwecken dienen. Daher weist die bekannte Vorrichtung eine Überwachungseinrichtung auf, mit der sich verschiedene Eigenschaften der abgesaugten Flüssigkeit ermitteln lassen. Die Daten der Überwachungseinrichtung geben dem behandelnden Arzt nicht nur Informationen über die Zusammensetzung der Flüssigkeit, sondern werden auch einer Regeleinheit zugeführt, um den Druck in der Injektionsleitung und die Durchflußmenge der injizierten Versorgungslösung auf bestimmten Werten zu halten.

Der Erfindung liegt die Aufgabe zugrunde, eine Vorrichtung zum Spülen von Körperhöhlen und Gewebeoberflächen zu schaffen, die eine bedarfsgerechte Anpassung der für eine optimale Spülung der Körperhöhle oder der Gewebefläche erforderlichen Spülflüssigkeitsmenge erlaubt, bei gleichzeitig hoher Bedienfreundlichkeit.

Die Lösung dieser Aufgabe erfolgt durch die Merkmale des Anspruches 1.

Die erfindungsgemäße Vorrichtung ermittelt durch ihre Sensoreinrichtung in der zweiten, die Auslaufleitung bildende Leitung, eine Veränderung der Spülflüssigkeit nach dem Durchlauf durch den Körper, die ein Maß für die Verschmutzung der Spülflüssigkeit darstellt. Der sich ergebende Meßwert wird der Volumenstrom-Einstelleinrichtung in der ersten Leitung zugeführt, so daß die Volumenstrom-Einstelleinrichtung den Spülfluß in der ersten, die Zuleitung bildende Leitung so steuern kann, daß beispielsweise eine vorgegebene Eigenschaft der Spüllösung, wie insbesondere deren Trübung, eingehalten werden kann.

Dadurch ergibt sich der Vorteil einer bedarfsgerechten Spülung, die beispielsweise das Risiko der Bildung eines Blutgerinsels erheblich herabsetzt, wobei sich zusätzlich ergibt, daß eine Ersparnis von Spüllösung möglich ist.

Wie gesagt, dient die Vorrichtung zum Spülen von Körperhöhlen bzw. Gewebeoberflächen, wobei hiermit Oberflächen gemeint sind, die entweder bluten oder sonstige Stoffe abgeben. Als Beispiel seien hier die Spülung von Blase oder Nierenbecken, die Peritoneal-Lavage und die Spülung von Operationswunden angeführt.

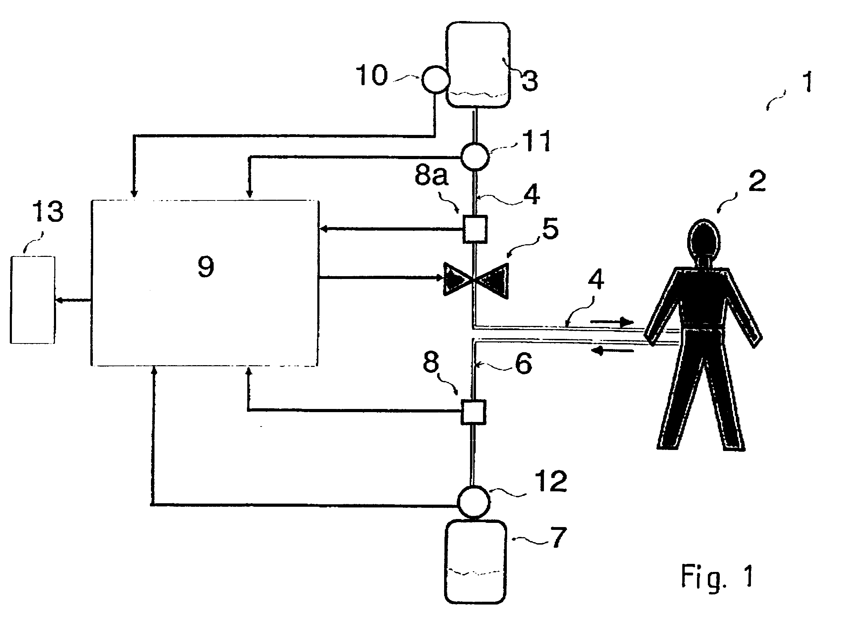

Der Behälter der erfindungsgemäßen Vorrichtung kann ein Beutel, eine Flasche oder auch eine Zentral-Versorgungsleitung sein. Als Leitung zum Körper ist vorzugsweise ein permanent gesetztes Endoskop vorgesehen, das über einen Spülkanal und einen Ableitungskanal verfügt. Dabei ist der Spülkanal mit dem Behälter für frische Spüllösungen bzw. Spülflüssigkeit verbunden, während der Ableitungskanal mit einem Abfluß bzw. einem Abfallbeutel verbunden sein kann (Fig. 1).

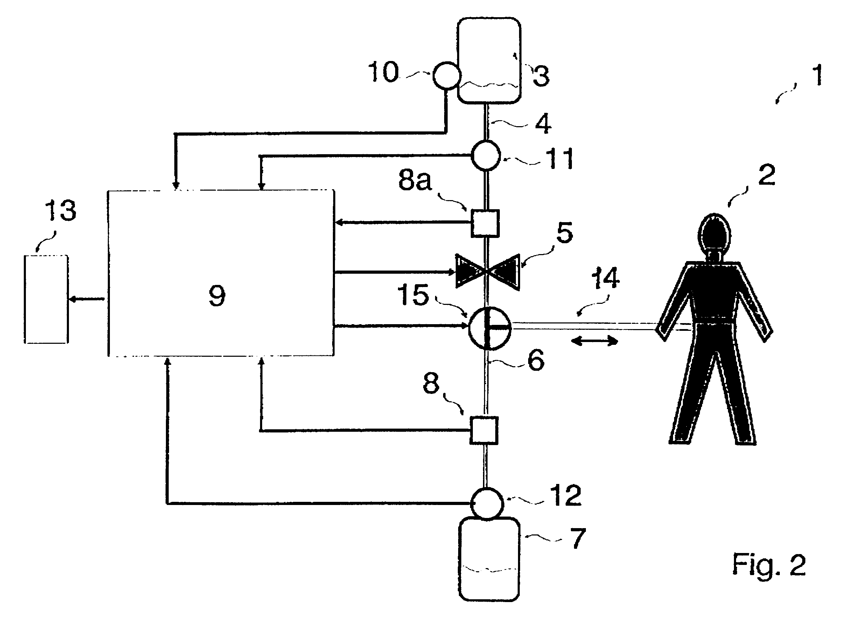

Grundsätzlich ist jedoch auch möglich, eine Leitung mit einem einzigen Zuführungskanal zu verwenden (Fig. 2). In diesem Falle wird intermittierend frische Flüssigkeit zugeführt bzw. verschmutste Flüssigkeit durch den einzigen Kanal abgeführt, entsprechend einem bestimmten Zeitschema. Dies kann manuell erfolgen über eine zusätsliche Volumenstrom-Einstelleinrichtung an oder unterhalb der Verzweigstelle. Andererseits kann aber auch vollautomatisch gemäß einem Programm frische Spülflüssigkeit zugeführt werden bzw. verschmutste Spüllösungen abgegeben werden, wobei sowohl die Zeitintervalle zwischen zwei Zuführschritten als auch der zugeführte Volumenstrom vorgegeben werden können.

Die Unteransprüche haben vorteilhafte Weiterbildungen der Erfindung zum Inhalt.

Als eine besonders bevorzugte zu ermittelnde Veränderung der Spülflüssigkeit ist deren Trübung anzusehen, da diese auf besonders einfache Art und Weise mittels einer optischen Detektoreinrichtung erfaßt werden kann.

Grundsätzlich ist es jedoch auch möglich, andere Veränderungen der Spülflüssigkeit wie insbesondere deren Farbe oder Leitfähigkeit als Maß für den Verschmutzungsgrad heranzuziehen, der über eine geeignete Sensoreinrichtung ermittelt werden kann.

Wie gesagt, ist als eine besonders bevorzugte Ausführungsform für die Sensoreinrichtung eine optische Detektoreinrichtung anzusehen. Diese kann bei einer besonders bevorzugten Ausführungsform zumindest eine lichtaussendende Diode (LED) aufweisen. Auf der der LED gegenüberliegenden Seite der zweiten Leitung wird bei einer derartigen Sensoreinrichtung ein Fotodetekor angeordnet, der den durch die getrübte Spülflüssigkeit hindurch verlaufenden Lichtstrahl empfängt und an eine Auswerteinrichtung weiterleitet.

Mit der erfindungsgemäßen Vorrichtung ist es weiterhin möglich, einen Soll-Ist-Wert-Vergleich in einer Regeleinheit vorzunehmen, dessen Ergebnis dann zur Regelung der Volumenstrom-Einstelleinrichtung herangezogen wird. Beispielsweise kann vom behandelnden Arzt der Trübungsgrad als Sollwert vorgegeben werden, bei dessen Überschreiten die Volumenstrom-Einstelleinrichtung solange geöffnet wird, bis der Sollwert wieder unterschritten ist. Daraufhin kann die Volumenstrom-Einstelleinrichtung gedrosselt oder vollständig geschlossen werden.

Im kontinuierlichen Betrieb gemäß Fig. 1 empfiehlt sich jedoch sicherzustellen, daß immer ein Minimalstrom an Spülflüssigkeit durch die Zuführungsleitung gelangt, der üblicherweise etwa ein Tropfen pro 2 Sekunden beträgt. Hierdurch wird sichergestellt, daß die in der zweiten Leitung angeordnete Sensoreinrichtung eine ausreichende Flüssigkeitsmenge, zur Bestimmung beispielsweise des Trübungsgrades, erhält.

In der erfindungsgemäßen Vorrichtung kann der gesamte Regelvorgang von einer Regeleinheit gesteuert werden, der die von der Sensoreinrichtung ermittelten Meßwerte verarbeitet und die Betätigungseinrichtung entsprechend ansteuert.

Wie eingangs bereits erwähnt, kann die Volumenstrom-Einstelleinrichtung als Pumpe, vorzugsweise als übliche Schlauchpumpe ausgebildet sein, bei der es möglich sein sollte, eine Minimalfördergeschwindigkeit zur Einstellung eines Minimalförderstromes für die Spüllösung einzustellen.

Ferner kann eine vorzugsweise motorisch betriebene Schlauchklemme als Volumenstrom-Einstelleinrichtung eingesetzt werden, die den Zulauf der Spüllösung in den Patienten in Abhängigkeit vom jeweils ermittelten Meßwert regelt. Auch eine derartige Schlauchklemme kann bei einer besonders bevorzugten Ausführungsform mit einer Einstellanordnung für einen Minimalfluß der Spüllösung versehen sein.

Die Volumenstrom-Einstelleinrichtung kann sowohl als Pumpe, als auch als Ventil eine stufenlose Veränderung des Volumenstroms bewirken, oder aber zwischen zwei oder mehr festen Einstellungen pendeln (z.B. im Rahmen einer Zweitpunktregelung).

Bei weiteren bevorzugten Ausführungsformen ist es möglich, einen Sensor für das Spüllösungsende, einen Sensor für ein Restvolumen der Spüllösung und für eine Spülflußunterbrechnung vorzusehen.

Der Restvolumensensor ist hierbei am Spüllösungsbehälter angeordnet, während der vorzugsweise als optischer Detektor ausgebildete Sensor für das Spüllösungsende in der ersten, die Zuleitung bildende Leitung angeordnet wird. Die Informationen des optischen Detektors für das Spüllösungsende können z.B. einen Personalruf auslösen. Da aber eine gewisse Zeit vergeht, bis das Personal den Spüllösungsvorrat erneuert hat, ist es bei einer weiteren bevorzugten Ausführungsform vorzusehen, bereits vor dem Spüllösungsende einen Voralarm auszulösen. Hierfür kann der Restvolumensensor eingesetzt werden. Ein derartiger Sensor kann als optischer Sensor oder auch als Waage o.dgl. ausgebildet sein.

Der Sensor für Spülflußunterbrechung ist bei einer weiteren bevorzugten Ausführungsform am Auslauf der Vorrichtung angeordnet. Dieser Sensor detektiert, ob ein ordnungsgemäßer Abfluß gewährleistet ist, um einen Druckaufbau z.B. in der zu spülenden Blase zu vermeiden. Dieser Umstand kann eintreten, wenn der Auslauf z.B. durch Gewebereste oder Blutgerinsel verstopft wird, oder aber der Auslaufschlauch geknickt wird. Weitere Einzelheiten, Merkmale und Vorteile der Erfindung ergeben sich aus nachfolgender Beschreibung von Ausführungsbeispielen anhand der Zeichnung.

Es zeigt:
- Fig. 1: eine schematisch stark vereinfachte Darstellung einer ersten Ausführungsform der erfindungsgemäßen Vorrichtung,
- Fig. 2: eine der Fig. 1 entsprechenden Darstellung einer zweiten Ausführungsform, für diskontinuierlichen Betrieb.

In Fig. 1 ist eine erste Ausführungsform der erfindungsgemäßen Vorrichtung 1 dargestellt, die zur Spülung von Körperhöhlen und Gewebeoberflächen eines Patienten 2, beispielsweise bei Operationen dient.

Die Vorrichtung 1 weist hier einen, eine Spüllösung enthaltenden Behälter 3 auf, der mit einer ersten Leitung 4, bespielsweise in Form eines Schlauches strömungsverbunden ist.

In der ersten Leitung 4 ist eine Volumenstrom-Einstelleinrichtung 5 angeordnet, die bei Betätigung den Spülfluß beeinflussen kann.

Die erste Leitung 4 führt zu der zu spülenden Körperhöhle (z.B. Blase) oder Gewebeoberfläche (z.B. Wunde). Die Ableitung der Spüllösung erfolgt über die Leitung 6 welche in dem Auslaß 7, beispielsweise einem Abfallbeutel, mündet.

Das in Strömungsrichtung gelegene Ende der Leitung 4 kann mit der vom Körper wegführenden Leitung 6 verbunden sein, beispielsweise in einem doppellumigen Katheter.

Die schematische vereinfachte Darstellung der Fig. 1 verdeutlicht, daß in der zweiten Leitung 6 eine Sensoreinrichtung 8 angeordnet ist. Die Sensoreinrichtung 8 dient dazu, eine meßbare Verhänderung der nach dem Durchlauf durch das Körpergefäß gebrauchten Spüllösung zu ermitteln. Diese meßbare Veränderung kann beispielsweise die Trübung, die Leitfähigkeit oder die Farbe der Spüllösung sein. Entsprechend der zu ermittelnden Veränderung ist die Sensoreinrichtung 8 ausgebildet, so daß sie dazu in der Lage ist, die jeweiligen die Veränderung wiedergebenen Meßwerte zu ermitteln. Vorzugsweise ist die Sensoreinrichtung 8 ein optischer Detektor, der zur Ermittlung des Trübungsgrades der Spüllösung eine LED und einen Fotodetektor auf gegenüberliegenden Seiten der vorzugsweise als Schlauch ausgebildeten Leitung 6 aufweist. Es ist selbstverständlich, das je nach der zu ermittelnden Veränderung die zweite Leitung 6 gegebenenfalls durchsichtig ausgebildet ist.

Wie Fig. 1 ferner verdeutlicht, ist die Sensoreinrichtung 8 mit einer schematisch vereinfacht dargestellten Regeleinheit 9 verbunden, so daß das Meßwertergebnis der Sensoreinrichtung 8 an die Regeleinheit 9 angelegt werden kann. Die Regeleinheit 9 wiederum steuert entsprechend dem Meßergebnis die Volumenstrom-Einstelleinrichtung 5 an, so daß diese einen optimalen Spülfluß unter Berücksichtigung der ermittelten Meßwerte einregeln kann.

Zur Erhöhung der Meßgenauigkeit kann in der Leitung 4 ein dem Sensor 8 entsprechender zweiter Sensor 8a angeordnet sein, und die Differenz der Meßergebnisse dieser beiden Sensoren von der Regeleinheit 9 zur Steuerung der Volumenstrom-Einstelleinrichtung 5 herangezogen werden. Diese Ausführungsform hat darüberhinaus den Vorteil, daß eine Kalibrierung der Regeleinheit 9 bei Verwendung unterschiedlicher Lösungen entfallen kann.

Die Volumenstrom-Einstelleinrichtung 5 dieser Ausführungsform ist als Schlauchklemme ausgebildet.

Im Ergebnis wird bei dieser Ausführungsform die Volumenstrom-Einstelleinrichtung 5 entsprechend dem Soll-Ist-Wert-Vergleich der Regeleinheit 9 betätigt, um den gewünschten Spülfluß optimal und in geeigneter Weise einstellen zu können.

Die dargestellte Ausführungsform der erfindungsgemäßen Vorrichtung gemäß Fig. 1 weist ferner einen am Behälter 3 angebrachten Sensor 10 für Restvolumen, sowie einen Detektor 11 für das Spüllösungs-Ende auf, der in der ersten Leitung 4 stromab des Behälters 3 angeordnet ist.

In der zweiten Leitung 6 ist ein Sensor 12 für Spülfluß-Unterbrechnung angeordnet.
Die Ausgänge der Sensoren 10, 11, 12 sind bei der dargestellten Ausführungsform über Regeleinheit 9 an einen akustischen und/oder optischen Signalgeber 13 angelegt.

In einer anderen Ausführungsform können die Ergebnisse der Sensoren auch unabhängig von Regeleinheit 9 verarbeitet werden.

In Fig. 2 ist eine zweite Ausführungsform der erfindungsgemäßen Vorrichtung dargestellt, bei der alle übereinstimmenden Teile mit den gleichen Bezugszeichen wie in Fig. 1 versehen sind.

Der Unterschied zu Fig. 1 besteht darin, daß hier die zuführende Leitung 4 und die Ableitung 6 bereits vor dem Körper zu einem Kanal 14 zusammengeführt sind. In der dargestellten Ausführungsform sind die drei Leitungen über ein Drei-Wege-Ventil 15 verbunden, der von der Regeleinheit 9 betätigt wird. Denselben Effekt würde eine weitere Volumenstrom-Einstelleinrichtung in der Leitung 6 erzielen.
Die in Fig. 1 schematisch dargestellte Apparatur dient der Spülung von Operationswunden oder Körperhöhlen mit einer im Behälter 3 vorrätigen Spüllösung. Eine Volumenstrom-Einstelleinrichtung 5, die ihrerseits von einer Regeleinheit 9 angesteuert wird, stellt die Spülgeschwindigkeit oder die Spülfrequenz nach Bedarf des zu spülenden Gewebes ein. Die Regeleinheit 9 ermittelt diesen Bedarfswert über Sensor 8 oder im Vergleich der Meßergebnisse der Sensoren 8a in der ersten, den Zulauf bildenden Leitung 4, und 8 in der zweiten, den Ablauf bildenden Leitung 6. Der Sensor 8 reagiert auf chemisch-physikalische Veränderungen der durch den Körper geleiteten Spülflüssigkeit, wie z.B. Trübung durch Blutungen während oder nach operativer Behandlung, Veränderung des Proteingehaltes etc.. Auswertung des Soll-Ist-Wert-Verhältnisses, sowie Steuerung der Volumeneinstell-Einrichtung 5, vorzugsweise einer regelbaren Schlauchklemme, geschieht elektronisch in Regeleinheit 9. Verschiedene Zusatzsensoren, die für die Funktion der Vorrichtung nicht notwendig sind, jedoch die Sicherheit des Patienten erhöhen, dienen der Selbstkontrolle dieses Regelmechanismus' und lösen z.B. bei Spüllösungsmangel Warnsignale aus. Besteht die Zuleitung zum Körper nur aus einem Schlauch 14, wie schematisch in Fig. 2 gezeigt, erfolgt die Spülung nicht kontinuierlich, sondern intermittierend. Ein Drei-Wege-Ventil an der Verzweigung der drei Leitungen 4, 6 und 14 dient der Koordinierung des Zu- und Ablaufs der Spüllösung.

Die erfindungsgemäße Vorrichtung dient somit zur bedarfsgerechten Spülung von Wunden oder natürlichen Körperhöhlen, wie bei endoskopischer Behandlung, Blasenspülung oder Peretoneal-Lavage.

## Patentansprüche

1. Vorrichtung (1) zum Spülen von Körperhöhlen und Gewebeoberflächen von Patienten (2) mit
- einem Reservoir (3) für Spülflüssigkeit,
- einer vom Reservoir (3) abgehenden ersten Leitung (4), die zum Körper des Patienten führt und in der eine Volumenstrom-Einstelleinrichtung (5) angeordnet ist, die den Spülfluß beeinflußt,
- einer zweiten, vom Körper wegführenden Leitung (6), die in einen Auslaß (7) mündet,
- einer in der zweiten Leitung (6) angeordneten Sensoreinrichtung (8) zur Ermittlung einer Veränderung einer den Verschmutzungsgrad der gebrauchten Spüllösung charakteristischen Meßgröße, und
einer Regeleinheit (9), die den von der Sensoreinrichtung (8) ermittelten Ist-Wert der den Verschmutzungsgrad der gebrauchten Spüllösung charakterisierenden Meßgröße mit einem Soll-Wert vergleicht **dadurch gekennzeichnet,** daß die Regeleinheit (9) entsprechend dem Ist-Soll-Wert Vergleich die Volumenstrom-Einstelleinrichtung (5) ansteuert.

2. Vorrichtung nach Anspruch 1, **dadurch gekennzeichnet**, daß die Leitung (4) direkt zu der zu spülenden Oberfläche des Patienten führt.

3. Vorrichtung nach Anspruch 1, **dadurch gekennzeichnet**, daß die erste Leitung (4) bereits vor dem Körper mit der zweiten Leitung (6) zu einem einzigen zum Körper führenden Kanal (14) verbunden ist.

4. Vorrichtung nach Anspruch 3, **dadurch gekennzeichnet**, daß ein Drei-Wege-Ventil (15) die Strömungsrichtung in Kanal (14) alternierend ändert.

5. Vorrichtung nach Anspruch 3, **dadurch gekennzeichnet**, daß eine alternierende Strömungsrichtung in Kanal (14) durch eine zusätzliche Schlauchklemme in der zweiten Leitung (6) geregelt wird.

6. Vorrichtung nach einem der Ansprüche 1 bis 5, **dadurch gekennzeichnet**, daß die Sensoreinrichtung (8) Änderungen physikalischer Parameter der gebrauchten Spüllösung ermittelt.

7. Vorrichtung nach Anspruch 6, **dadurch gekennzeichnet**, daß die Sensoreinrichtung (8) die Trübung der gebrauchten Spüllösung ermittelt.

8. Vorrichtung nach Anspruch 6, **dadurch gekennzeichnet**, daß die Sensoreinrichtung (8) die Farbe der gebrauchten Spüllösung ermittelt.

9. Vorrichtung nach einem der Ansprüche 1 bis 5, **dadurch gekennzeichnet**, daß die Sensoreinrichtung (8) Änderungen chemischer Parameter der gebrauchten Spüllösung ermittelt.

10. Vorrichtung nach einem der Ansprüche 1 bis 9, **dadurch gekennzeichnet**, daß die Sensoreinrichtung (8) als optische Detektoreinrichtung ausgebildet ist.

11. Vorrichtung nach Anspruch 10, **dadurch gekennzeichnet**, daß die Sensoreinrichtung (8) zumindest eine lichtemittierende Quelle auf einer Seite der zweiten Leitung (6) und einen, auf der gegenüberliegenden Seite der Leitung (6) angeordneten, Fotodetektor aufweist.

12. Vorrichtung nach einem der Ansprüche 1 bis 11, **dadurch gekennzeichnet,** daß die Regeleinheit (9) den Ist-Wert aus dem vergleichenden Meßergebnis des Sensors (8) mit einem weiteren Sensor (8a) ermittelt, der in der Leitung (4) angeordnet ist.

13. Vorrichtung nach einem der Ansprüche 1 bis 12, **dadurch gekennzeichnet**, daß die Volumenstrom-Einstelleinrichtung (5) als Pumpe ausgebildet wird.

14. Vorrichtung nach einem der Ansprüche 1 bis 12, **dadurch gekennzeichnet**, daß die Volumenstrom-Einstelleinrichtung (5) als Ventil, vorzugsweise als Schlauchklemme, ausgebildet wird.

15. Vorrichtung nach Anspruch 13 oder 14, **dadurch gekennzeichnet**, daß die Volumenstrom-Einstelleinrichtung (5) eine Einstellanordnung für einen Minimalfluß der Spüllösung aufweist.

16. Vorrichtung nach einem der Ansprüche 1 bis 15, **dadurch gekennzeichnet**, daß der Behälter (3) mit einem Restvolumen-Sensor (10) versehen ist.

17. Vorrichtung nach einem der Ansprüche 1 bis 15, **dadurch gekennzeichnet**, daß in der ersten Leitung (4) ein vorzugsweise optischer Detektor (11) für Spülflußüberwachung vorgesehen ist.

18. Vorrichtung nach einem der Ansprüche 1 bis 17, **dadurch gekennzeichnet**, daß in der zweiten Leitung (6) ein Sensor (12) für Spülflußunterbrechung angeordnet ist.

19. Vorrichtung nach einem der Ansprüche 16, 17 oder 18, **dadurch gekennzeichnet**, daß die Sensoren (10), (11) oder (12) einen Signalgeber (13) ansteuern.

## Claims

1. Apparatus (1) for the irrigation of body cavities and tissue surfaces of patients (2) with
- a reservoir (3) for irrigation fluid,
- a first conduit (4) emanating from the reservoir (3) and leading to the patient's body, a volume stream adjusting device (5) affecting the irrigation flow being arranged in this conduit,
- a second conduit (6) leading away from the body and terminating into an outlet (7),
- a sensor device (8) being disposed in the second conduit (6) for determining a measurable change characteristic for the degree of soiling of the exhausted irrigation solution,
- a control unit (9) which compares the actual value of the measurable variable characterizing the degree of soiling of the exhausted irrigation solution determined by the sensor device 88) with a set value,
characterized in that the control unit (9) operates the volume stream adjusting device (5) according to the actual-set-value comparison.

2. Apparatus according to claim 1, characterized in that the conduit (4) leads directly to the patient's tissue surface to be irrigated.

3. Apparatus according to claim 1, characterized in that the first conduit (4) is joined already upstream of the body with the second conduit (6) into a single duct (14) leading to the body.

4. Apparatus according to claim 3, characterized in that a three-way valve (15) changes the flow direction in the duct (14) in alternating fashion.

5. Apparatus according to claim 3, characterized in that an alternating flow direction in the duct (14) is regulated by an additional hose clamp in the second conduit (6).

6. Apparatus according to one of claims 1 - 5, characterized in the sensor device (8) determines changes of physical parameters of the exhausted irrigation solution.

7. Apparatus according to claim 6, characterized in that the sensor device (8) determines the turbidity of the exhausted irrigation solution.

8. Apparatus according to claim 6, characterized in that the sensor device (8) monitors the colour of the exhausted irrigation solution.

9. Apparatus according to one of claims 1 - 5, characterized in that the sensor device (8) determines changes of chemical parameters of the exhausted irrigation solution.

10. Apparatus according to one of claims 1 - 9, characterized in that the sensor device (8) is an optical detector unit.

11. Apparatus according to claim 10, characterized in that the sensor device (8) utilizes at least one light-emitting source on one side of the second conduit (6) and a photodetector located on the opposite side of the conduit (6).

12. Apparatus according to one of claims 1 - 11, characterized in that the control unit (9) determines the actual value from the comparative measured result of the sensor (8) with a further sensor (8a) located in the conduit (4).

13. Apparatus according to one of claims 1 - 12, characterized in that the volume stream adjusting device (5) is a pump.

14. Apparatus according to one of claims 1 - 12, characterized in that the volume stream adjusting device (5) is a valve preferably configured as a hose clamp.

15. Apparatus according to claim 13 or 14, characterized in that the volume stream adjusting device (5) comprises a setting arrangement for a minimum flow of irrigation solution.

16. Apparatus according to one of claims 1 - 15, characterized in that the container (3) is equipped with a residual volume sensor (10).

17. Apparatus according to one of claims 1 - 15, characterized in that a preferably optical detector (11) for irrigation flow monitoring is provided in the first conduit (4).

18. Apparatus according to one of claims 1 - 17, characterized in that a sensor (12) for irrigation flow interruption is arranged in the second conduit (6).

19. Apparatus according to one of claims 16, 17 or 18, characterized in that the sensors (10), (11), or (12) trigger a signal generator (13).

## Revendications

1. Dispositif (1) pour l'irrigation de cavités corporelles et de surfaces de tissu de patients (2), comprenant :
- un réservoir (3) pour le liquide d'irrigation,
- une première conduite (4) partant du réservoir (3), qui mène au corps du patient et dans laquelle est agencé un dispositif (5) de réglage du débit volumique gui agit sur le courant d'irrigation,
- une deuxième conduite (6) qui part du corps, qui débouche dans une sortie (7),
- un dispositif capteur (8) agencé dans la deuxième conduite (6), servant à détecter une variation d'une grandeur de mesure caractéristique du degré de souillure de la solution d'irrigation usée, et
- une unité de régulation (9) qui compare à une valeur de consigne la valeur réelle, captée par le dispositif capteur (8), de la grandeur de mesure qui caractérise le degré de souillure de la solution d'irrigation usée,
caractérisé
en ce que l'unité de régulation (9) pilote le dispositif (5) de réglage du débit volumique en fonction de la comparaison entre valeur réelle et valeur de consigne.

2. Dispositif selon la revendication 1, caractérisé en ce que la conduite (4) conduit directement à la surface du patient qu'il s'agit d'irriguer.

3. Dispositif selon la revendication 1, caractérisé en ce que la première conduite (4) est déjà reliée en amont du corps à la deuxième conduite (6) pour former un canal (14) unique conduisant au corps.

4. Dispositif selon la revendication 3, caractérisé en ce qu'un robinet à trois voies (15) modifie en alternance le sens de l'écoulement dans le canal (14).

5. Dispositif selon la revendication 3, caractérisé en ce qu'une direction d'écoulement alternée dans le canal (14) est réglée par une pince à tube additionnelle intercalée dans la deuxième conduite (6).

6. Dispositif selon une des revendications 1 à 5, caractérisé en ce que le dispositif capteur (8) capte des modifications de paramètres physiques de la solution d'irrigation usée.

7. Dispositif selon la revendication 6, caractérisé en ce que le dispositif capteur (8) capte la turbidité de la solution d'irrigation usée.

8. Dispositif selon la revendication 6, caractérisé en ce que le dispositif capteur (8) capte la couleur de la solution d'irrigation usée.

9. Dispositif selon une des revendications 1 à 5, caractérisé en ce que le dispositif capteur (8) capte des variations de paramètres chimiques de la solution d'irrigation usée.

10. Dispositif selon une des revendications 1 à 9, caractérisé en ce que le dispositif capteur (8) est constitué par un dispositif détecteur optique.

11. Dispositif selon la revendication 10, caractérisé en ce que le dispositif capteur (8) comprend au moins une source émettrice de lumière, sur un côté de la deuxième conduite (6), et un photodétecteur agencé sur le côté opposé de la conduite (6).

12. Dispositif selon une des revendications 1 à 11, caractérisé en ce que l'unité de régulation (9) capte la valeur réelle sur la base du résultat de mesures comparatives du capteur (8) avec un autre capteur (8a) qui est agencé dans la conduite (4).

13. Dispositif selon une des revendications 1 à 12, caractérisé en ce que le dispositif (5) de réglage du débit volumique est constitué par une pompe.

14. Dispositif selon une des revendications 1 à 12, caractérisé en ce que le dispositif (5) de réglage du débit volumique est constitué par un robinet, de préférence par une pince à tube.

15. Dispositif selon la revendication 13 ou 14, caractérisé en ce que le dispositif (5) de réglage du débit volumique présente un dispositif de réglage donnant un courant minimum de la solution d'irrigation.

16. Dispositif selon une des revendications 1 à 15, caractérisé en ce que le récipient (3) est muni d'un capteur (10) de volume résiduel.

17. Dispositif selon une des revendications 1 à 15, caractérisé en ce qu'un détecteur (11), de préférence optique, destiné à la surveillance du courant d'irrigation, est prévu dans la première conduite (4).

18. Dispositif selon une des revendications 1 à 17, caractérisé en ce qu'un capteur (12) pour l'interruption du courant d'irrigation est agencé dans la deuxième conduite (6).

19. Dispositif selon une des revendications 16, 17 ou 18, caractérisé en ce que les capteurs (10), (11) ou (12) pilotent un émetteur de signaux (13).
